# FASCICULE DE BREVET EUROPEEN

(11) **EP 1 858 481 B1**
(45) Date de publication et mention de la délivrance du brevet: **08.05.2019**
(21) Numéro de dépôt: 06726059.6
(22) Date de dépôt: 09.03.2006
(51) Int. Cl.: A61K 31/4409, A61K 31/167, A61K 31/137, A61K 9/00, A61P 17/12

(54) **COMPOSITIONS OPHTALMOLOGIQUES ET LEUR UTILISATION**
OPHTHALMOLOGISCHE ZUSAMMENSETZUNGEN UND IHRE VERWENDUNG
OPHTHALMOLOGIC COMPOSITIONS AND USE THEREOF

(30) Priorité: 09.03.2005 FR 0502357
(43) Date de publication de la demande: 28.11.2007
(73) Titulaire: Laboratoires THEA, 63100 Clermont-Ferrand (FR)
(72) Inventeur: KHATIB, Walid, F-20600 Bastia (FR)
(74) Mandataire: Cabinet Laurent & Charras
(86) Numéro de dépôt international: PCT/FR2006/000528
(87) Numéro de publication internationale: WO 2006/095095

(56) Documents cités:
- WO-A-01/34088
- WO-A-97/17989
- WO-A2-2004/010894
- US-A- 3 026 323
- US-A- 5 779 661
- US-A1- 2004 013 729
- US-B1- 6 218 428
- DATABASE BIOSIS [Online] BIOSCIENCES INFORMATION SERVICE, PHILADELPHIA, PA, US; 1976, HUSAROVA E S: "FUNCTIONAL STATE OF THE CILIATED EPITHELIUM UNDER THE EFFECT OF THE AEROSOL EPHATIN" XP002383518 Database accession no. PREV197764070970 & FARMATSEVTYCHNYI ZHURNAL (KIEV), vol. 31, no. 4, 1976, pages 67-69, ISSN: 0367-3057
- MATHER R ET AL: "The effect of cataract surgery on ocular levels of topical moxifloxacin" AMERICAN JOURNAL OF OPHTHALMOLOGY, OPHTHALMIC PUBL., CHICAGO, IL,, US, vol. 138, no. 4, octobre 2004 (2004-10), pages 554-559, XP004646323 ISSN: 0002-9394
- MILLER S A ET AL: "SYSTEMIC REACTION TO SUBCONJUNCTIVAL PHENYLEPHRINE" CANADIAN JOURNAL OF OPHTHALMOLOGY, XX, XX, vol. 13, no. 4, 1978, pages 291-293, XP009055684 ISSN: 0008-4182
- BEHNDIG A ET AL: "Evaluation of surgical performance with intracameral mydriatics in phacoemulsification surgery" ACTA OPHTHALMOLOGICA SCANDINAVICA, HVIDOVRE, DK, vol. 82, no. 2, 2004, pages 144-147, XP002350415 ISSN: 1395-3907
- KEEMBIYAGE RANASIRI DAYAWANSA ET AL: "Tachycardia and myocardial ischaemia following subconjunctival injection of mydricaine (number 02) for vitrectomy procedure." CLINICAL & EXPERIMENTAL OPHTHALMOLOGY. FEB 2005, vol. 33, no. 1, février 2005 (2005-02), pages 105-106, XP002383514 ISSN: 1442-6404
- REAH G ET AL: "Peribulbar anaesthesia using a mixture of local anaesthetic and vecuronium." ANAESTHESIA. JUN 1998, vol. 53, no. 6, juin 1998 (1998-06), pages 551-554, XP002383515 ISSN: 0003-2409
- ONG-TONE LINDSAY: "Use of a wick to deliver preoperative mydriatics for cataract surgery." JOURNAL OF CATARACT AND REFRACTIVE SURGERY. NOV 2003, vol. 29, no. 11, novembre 2003 (2003-11), pages 2060-2062, XP002383585 ISSN: 0886-3350
- BLACK E H ET AL: "Necessity of the Honan intraocular pressure reducer in cataract surgery using topical anesthesia" JOURNAL CATARACT AND REFRACTIVE SURGERY, AMERICAN SOCIETY OF CATARACT AND REFRACTIVE, US, vol. 25, no. 2, février 1999 (1999-02), pages 223-226, XP004745881 ISSN: 0886-3350
- PAGANELLI F ET AL: "A single intraoperative sub-Tenon's capsule triamcinolone acetonide injection for the treatment of post-cataract surgery inflammation" OPHTHALMOLOGY, J. B. LIPPINCOTT CO., PHILADELPHIA, PA, US, vol. 111, no. 11, novembre 2004 (2004-11), pages 2102-2108, XP004670067 ISSN: 0161-6420
- MATHALONE N ET AL: "Long-term intraocular pressure control after clear corneal phacoemulsification in glaucoma patients" JOURNAL CATARACT AND REFRACTIVE SURGERY, AMERICAN SOCIETY OF CATARACT AND REFRACTIVE, US, vol. 31, no. 3, mars 2005 (2005-03), pages 479-483, XP004848152 ISSN: 0886-3350
- APT L ET AL: "PUPILLARY DILATATION WITH SINGLE EYEDROP MYDRIATIC COMBINATIONS" AMERICAN JOURNAL OF OPHTHALMOLOGY, OPHTHALMIC PUBL., CHICAGO, IL,, US, vol. 89, no. 4, 1980, pages 553-559, XP009055683 ISSN: 0002-9394

## Description

La présente demande concerne le domaine de la thérapeutique et plus particulièrement le domaine de l'ophtalmologie.

Il est décrit plus particulièrement de nouvelles compositions pharmaceutiques destinées au traitement de la douleur et/ou des lésions de l'oeil au cours d'une intervention chirurgicale ou physique ou préalablement à une intervention sur l'oeil.

Il est décrit des compositions à base d'un agent parasympatholytique, d'une substance adrénergique et avantageusement d'un anesthésique de contact, dans le cadre d'un traitement pré-opératoire de l'oeil en vue de l'opération chirurgicale de la cataracte ou d'un traitement de la rétine au laser. Ces principes actifs sont destinés à être administrés simultanément sur ou dans l'oeil.

Plus précisément, l'invention concerne une composition pharmaceutique pour utilisation dans la préparation de l'oeil avant une intervention chirurgicale ou au cours de l'intervention chirurgicale, renfermant une association d'un agent parasympatholytique, d'un agent sympathomimétique et d'un anesthésique de contact, caractérisée en ce que :
- la composition comprend :
   - une concentration en tropicamide, en tant qu'agent parasympatholytique, comprise entre 0,015 et 0,025% ;
   - une concentration en lidocaïne ou un de ses sels, en tant qu'anesthésique de contact, comprise entre 0,75 et 1,25% ; et
   - une concentration en phényléphrine, en tant qu'agent sympathomimétique, comprise entre 0,2 et 0,4% ;
- la composition est administrée par injection intracamérulaire ;
- l'intervention chirurgicale est une opération de la cataracte.

L'agent parasympatholytique peut être un agent mydriatique et de préférence, un dérivé de l'acide tropique comme un ester de l'acide tropique ou bien encore un amide de l'acide tropique ou un amide de l'acide cyclohexyl ou cyclopentylacétique.

Parmi ces agents parasympatholytiques, on nommera plus particulièrement la cyclodrine, l'eucatropine, l'homatropine ainsi que l'atropine (ester benzylique de l'acide tropique), la N-méthylhyoscine et surtout le tropicamide (N-éthyl 2-phényl N-4 pyridyl méthyl hydracrylamide ou pyridyl méthyl benzène acétamide).

Parmi les anesthésiques de contact, on peut citer plus particulièrement la butocaïne, la prilocaïne, la procaïne, la novocaïne, la tétracaïne, l'ambucaïne, l'amylocaïne, la bupivacaïne, la carticaïne, la butoxycaïne, la formocaïne, la mévipacaïne, l'étidocaïne, la prilocaïne, l'orthocaïne, l'oxybuprocaïne ou benoxinate, et principalement la lidocaïne ou diéthylamino 2,6 diméthyl acètanilide, ou l'un de ses sels.

Parmi les substances adrénergiques, on peut citer plus particulièrement la noradrénaline, la phenyléphrine, l'isoprénaline, la dipivéfrine, la dimétrophine, la norfénéfrine, la pholédrine ou l'octédrine.

Des combinaisons de ce type ont déjà été décrites dans la littérature.

Le document US 6,218,428 enseigne des compositions ophtalmiques pouvant être utilisées dans le cadre de l'opération de la cataracte et contenant de 0,01 % à 15 % d'un agent parasympatholytique ou sympathomimétique.

Le document US 2004/013729 décrit des compositions pour administration topique comprenant 0,05 % de propacaïne, 0,48 % de phényléphrine et 0,25 % de tropicamide. Il est décrit des compositions améliorées pour dilater la pupille de l'oeil, utilisées par voie topique, et proposé d'utiliser un polymère viscoélastique pour augmenter le temps de résidence de la composition sur la cornée, et améliorer ainsi la pénétration des principes actifs.

Dayawansa et al. (Clinical & Expérimental Ophthalmomogy, 2005, 33(1), 105-106 ) ont décrit une composition destinée à une injection sous-conjonctivale comprenant 1,3 mg d'atropine sulfate, 0,12 mg d'adrénaline et 8,4 mg de procaïne hydrochloride, dans un volume de 0,4 mL.

Reah et al. (Anesthesia, 1998, 53(6), 551-4) ont décrit une composition anesthésique utilisée pour la chirurgie oculaire, notamment la chirurgie de la cataracte, par injection péribulbaire, comprenant 1 % de lidocaïne, 1:400 000 d'adrénaline et 0,375 % de bupivacaïne. Préalablement à l'administration de cette composition anesthésique, les patients opérés de la cataracte recevraient des agents mydriatiques.

Le document WO2004/010894 décrit des solutions destinées à être administrées localement dans l'oeil de manière continue, et comprenant des principes actifs inhibant l'inflammation, inhibant la douleur, affectant la dilatation de la pupille et/ou diminuant la pression intraoculaire, présents « en faibles concentrations ». Ce document enseigne que dans le cadre d'une opération de la cataracte, la présence d'analgésique n'est pas indispensable.

La publication Behndig A et al. (Acta Ophthalmologica Scandinavica (2004) 82(2) 144-147) décrit l'utilisation d'une composition contenant 0,1% de cyclopentolate, 1,5% de phényléphrine et 1% de lidocaïne, destinée à l'administration intracamérulaire dans le cadre d'une intervention chirurgicale par phacoémulsification.

Dans cette publication, les résultats obtenus par injection intracamérulaire des mydriatiques ont été comparés à ceux obtenus par application topique. Les effets, et surtout les effets secondaires, ne sont pas différents. Il apparaît important dans cette technique d'éviter les effets généraux liés à l'administration d'epinéphrine ou à l'administration intracamérulaire de lidocaïne.

Une autre publication (L. Apt et al. Am J. of Ophthalmology 89(4) (1980) 553-559) décrit l'utilisation de compositions ophtalmologiques contenant du tropicamide (0,5% ou 1%) ou du cyclopentolate (0,5%) associé à de la phényléphrine 2,5%, en instillation dans l'oeil, à raison d'une goutte dans chaque oeil. Dans certaine cas, l'instillation de la combinaison des deux agents mydriatiques est précédée par l'application topique d'une goutte d'anesthésiant proparacaïne 0,5%. L'association des trois principes actifs n'est toutefois pas envisagée.

En outre, S.A. Miller et W.F. Mieler ont montré (Canad. J. Ophtal 13 (1978) 291-293) les effets systémiques résultant de l'injection sous-conjonctivale d'une association de phényléphrine à très forte concentration (3,3%), de cocaïne (anesthésique local à 1,3%) et d'atropine (0,3%), se traduisant notamment par une augmentation de la pression sanguine. Cette augmentation a été suivie par de l'hypotension, de l'oedème pulmonaire et une ischémie endocardiaque. Les auteurs concluent en insistant sur les effets dangereux, sur la pression sanguine, de la phényléphrine appliquée à l'oeil.

Ces références, considérées comme l'art antérieur le plus proche, montrent bien la nécessité de conjuguer efficacement une action pharmacologique importante et une absence quasiment totale d'effets généraux résultant de la résorption au niveau de l'oeil de médicaments ayant des effets systémiques.

Les compositions selon l'invention présentent un intérêt majeur en injection intracamérulaire, pour la préparation de l'oeil avant une intervention chirurgicale ou au cours de l'intervention chirurgicale, dans le traitement chirurgical de la cataracte. Le volume de la chambre intracamérulaire n'excédant pas 300 µl chez l'homme, le volume de composition injectée est préférentiellement inférieur ou égal à 200 µl, notamment compris entre 50 et 200 µl.

Il est important de noter que les compositions selon l'invention sont en mesure d'assurer une anesthésie très rapide et aussi une dilatation quasi instantanée, importante et durable de la pupille qui permet d'effectuer l'examen de la pupille et de procéder à l'intervention chirurgicale dans les plus brefs délais. Il faut souligner également que l'anesthésie réalisée est de longue durée, de sorte que les sujets ont peu à souffrir de l'intervention chirurgicale qu'ils subissent. Une telle association présente l'avantage de renforcer l'effet mydriatique du dérivé parasympatholytique, sans toutefois manifester un effet trop prolongé qui entraîne une diminution de la vision se caractérisant essentiellement par une mydriase trop prolongée. Les effets de la mydriase se traduisent principalement par des troubles de la vision et des troubles de l'équilibre.

Il est donc apparu que les rapports entre les principes actifs devaient être déterminés avec précision.

Les compositions selon l'invention se distinguent de celles décrites dans l'art antérieur par une concentration en agent sympathomimétique, par exemple en phényléphrine, nettement abaissée. La concentration en agent parasympatholytique est également diminuée.

Il est décrit des compositions pharmaceutiques renfermant de 0,001 à 0,6% d'agent parasympatholytique, de 0,04 à 0,5% d'agent sympathomimétique, et éventuellement de 0,2 à 3% d'agent anesthésique de contact.

Une composition préférée renferme de 0,01 à 0,5% d'agent parasympatholytique défini comme précédemment, de 0,5 à 1,5% d'anesthésique de contact et de 0,1 à 0,4% d'agent sympathomimétique.

Une composition ophtalmologique utilisée dans le cadre de l'invention renferme de 0,015 à 0,025% d'un agent parasympatholytique, en l'occurrence le tropicamide, de 0,75% à 1,25 % de lidocaïne et de 0,2 à 0,4% de phényléphrine dans une seule préparation. Une telle formulation est injectée dans la chambre antérieure de l'oeil avant un traitement chirurgical de la cataracte. Pour cela, une incision est pratiquée au moment de l'intervention dans la chambre antérieure de l'oeil et la composition y est injectée. Cette préparation de l'oeil permet simultanément de provoquer une mydriase et une anesthésie locale, permettant l'extraction ultérieure du cristallin dans des conditions favorables. Dans ce cas précis, une injection unique d'un volume de la préparation compris entre 0,05 et 0,2 ml permet d'obtenir l'effet recherché.

Il est également décrit que dans le cas d'une intervention chirurgicale au laser, les concentrations des compositions pharmaceutiques instillées sont avantageusement adaptées. En particulier, les concentrations en agent parasympatholytique et en agent anesthésique de contact seront sensiblement plus élevées, situés dans les valeurs hautes de la gamme de concentrations la plus large. Ainsi et de manière préférentielle, les concentrations en agent parasympatholytique seront de l'ordre de 0,1 à 0,25% et la concentration en agent anesthésique de contact sera de 2 à 3%. De telles préparations étant destinées à un usage unique, l'utilisation d'agents conservateurs n'est pas forcément nécessaire. Les préparations décrites ne contiendront donc éventuellement pas d'agent conservateur. Dans ce cas de figure, le mode d'administration peut également être différent, en procédant à l'instillation de la préparation sur l'oeil traité, de deux à quatre fois, à une minute d'intervalle. Ainsi, la sédation de la douleur est assurée d'une manière plus immédiate et plus prolongée.

Selon l'invention, les trois principes actifs sont dissous ou dispersés dans un véhicule aqueux, avantageusement stérile. Les compositions selon l'invention peuvent se présenter sous forme liquide ou solide, par exemple sous forme de collyres, de récipients unidoses, de préparations instillables prêtes à l'emploi ou sous forme de lyophilisats à reconstituer avec un solvant aqueux au moment de l'emploi.

Les compositions selon l'invention peuvent également se présenter sous forme mixte comme par exemple un lyophilisat d'un des principes actifs que l'on reconstitue en ajoutant une solution aqueuse contenant les autres principes actifs. Il est également possible de disposer dans un flacon un des principes actifs solides, surmonté d'une membrane frangible au-dessus de laquelle se trouve la solution des autres principes actifs.

L'invention sera mieux définie à l'aide des exemples qui figurent ci-après :

### Exemple 1

Préparation (forte dose) à usage unique pour injection intracamérulaire préalablement à une intervention chirurgicale :
Tropicamide 0,00024 g
Phényléphrine (chlorhydrate) 0,0038g
Lidocaïne (chlorhydrate) 0,00917 g
Eau pour préparation injectable QSP pour un flacon de 1 ml à usage unique

La préparation selon l'invention est destinée à être administrée par injection dans la chambre antérieure de l'oeil (zone intracamérulaire).

Ladite préparation pourra être présentée dans un conditionnement à usage unique permettant de manipuler facilement et en toute sécurité, puis d'administrer au patient, une quantité de liquide inférieure à 1 ml, typiquement comprise entre 50 et 200 µl.

### Exemple 2

Préparation (dose habituelle) à usage unique pour injection intracamérulaire préalablement à une intervention chirurgicale :
Tropicamide 0,00016 g
Phényléphrine (chlorhydrate) 0,00258g
Lidocaïne (chlorhydrate) 0,00943 g
Eau pour préparation injectable QSP pour un flacon de 1 ml à usage unique

La préparation est utilisée dans les mêmes conditions qu'à l'exemple 1.

### Exemple 3 (hors invention)

Collyre sans conservateur au tropicamide pour utilisation préalablement à une intervention chirurgicale :
Tropicamide 0,05 g
Norépinephrine (chlorhydrate) 0,02g
Lidocaïne (chlorhydrate) 0,12 g
Eau purifiée stérile 10 ml

Ladite préparation sera présentée dans un conditionnement à usage unique.

### Exemple 4 (hors invention)

Collyre sans conservateur pour administration pré-laser :
Tropicamide 0,0015 g
Phényléphrine 0,030 g
Bupivacaïne(chlorhydrate) 0,25 g
Eau purifiée stérile QSP 10 ml

Ladite préparation sera présentée dans un conditionnement à usage unique.
Ce collyre convient particulièrement pour le traitement de la douleur au décours d'un traitement au laser.

Les collyres selon l'invention sont à instiller sur l'oeil, à raison de trois fois une goutte au cours d'une même journée.

## Revendications

1. Composition pharmaceutique pour utilisation dans la préparation de l'oeil avant une intervention chirurgicale ou au cours de l'intervention chirurgicale, renfermant une association d'un agent parasympatholytique, d'un agent sympathomimétique et d'un anesthésique de contact, ***caractérisée* en ce que** :
• la composition comprend :
- une concentration en tropicamide, en tant qu'agent parasympatholytique, comprise entre 0,015 et 0,025% ;
- une concentration en lidocaïne ou un de ses sels, en tant qu'anesthésique de contact, comprise entre 0,75 et 1,25% ; et
- une concentration en phényléphrine, en tant qu'agent sympathomimétique, comprise entre 0,2 et 0,4% ;
• la composition est administrée par injection intracamérulaire ;
• l'intervention chirurgicale est une opération de la cataracte.

2. Composition pharmaceutique pour son utilisation selon la revendication 1, ***caractérisée* en ce que** la composition pharmaceutique se présente sous forme de solution ou de suspension aqueuse, ou de flacon unidose.

3. Préparation à usage unique pour injection intracamérulaire préalablement à une intervention chirurgicale constituée de:
Tropicamide : 0,00024 g
Chlorhydrate de phényléphrine : 0,0038g
Chlorhydrate de lidocaïne : 0,00917 g
Eau pour préparation injectable QSP pour un flacon de 1 ml à usage unique.

4. Préparation à usage unique pour injection intracamérulaire préalablement à une intervention chirurgicale constituée de:
Tropicamide : 0,00016 g
Chlorhydrate de phényléphrine : 0,00258g
Chlorhydrate de lidocaïne : 0,00943 g
Eau pour préparation injectable QSP pour un flacon de 1 ml à usage unique.

## Patentansprüche

1. Pharmazeutische Zusammensetzung zur Verwendung bei der Vorbereitung des Auges für einen chirurgischen Eingriff oder während des chirurgischen Eingriffs, die eine Kombination aus einem Parasympatholytikum, einem Sympathomimetikum und einem Kontaktanästhetikum enthält, **dadurch gekennzeichnet, dass**
- die Zusammensetzung umfasst:
- eine Konzentration an Tropicamid als parasympatholytischer Wirkstoff, zwischen 0,015 und 0,025%;
- eine Konzentration an Lidocain oder eines seiner Salze als Kontaktanästhetikum zwischen 0,75 und 1,25%; und
- eine Konzentration an Phenylephrin als sympathomimetischer Wirkstoff zwischen 0,2 und 0,4% ;
- die Zusammensetzung durch intrakamerale Injektion verabreicht wird:
- es sich beim chirurgischen Eingriff um eine Kataraktoperation handelt.

2. Pharmazeutische Zusammensetzung zur Verwendung nach Anspruch 1, **dadurch gekennzeichnet, dass** die pharmazeutische Zusammensetzung die Form einer wässrigen Lösung oder Suspension oder eines Einzeldosisflakons hat.

3. Zubereitung zum Einmalgebrauch zur intrakameralen Injektion vor einem chirurgischen Eingriff, bestehend aus:
Tropicamid: 0,00024 g,
Phenylephrinchlorhydrat: 0,0038g,
Lidocainchlorhydrat: 0,00917 g,
Wasser für die injizierbare Zubereitung Q.S. für einen 1 ml-Flakon zum Einmalgebrauch.

4. Zubereitung zum Einmalgebrauch zur intrakameralen Injektion vor einem chirurgischen Eingriff, bestehend aus:
Tropicamid: 0,00016 g,
Phenylephrinchlorhydrat: 0,00258g,
Lidocainchlorhydrat: 0.00943 g,
Wasser für die injizierbare Zubereitung Q.S. für einen 1 ml-Flakon zum Einmalgebrauch.

## Claims

1. A pharmaceutical composition for use in preparing the eye before a surgical operation or during the surgical operation, containing a combination of a parasympatholytic agent, a sympathomimetic agent and a local anesthetic, ***characterized in that** :*
• the composition comprises:
- a concentration of tropicamide, as a parasympatholytic agent, between 0.015 and 0.025%;
- a concentration of lidocaine or a salt thereof, as a local anesthetic, between 0.75 and 1.25%; and
- a concentration of phenylephrine, as a sympathomimetic agent, between 0.2 and 0.4%;
• the composition is administered by intracameral injection ;
• the surgical operation is a cataract operation.

2. A pharmaceutical composition for its use according to claim 1, ***characterized in that*** the pharmaceutical composition is in the form of an aqueous solution or suspension, or of a single-dose bottle.

3. Single-use preparation for intracameral injection prior to a surgical operation consisting of:
Tropicamide: 0.00024g
Phenylephrine hydrochloride: 0.0038g
Lidocaine hydrochloride: 0.00917g
Water for injectable preparation Q.S. for a 1 ml bottle for single use.

4. Single-use preparation for intracameral injection prior to a surgical operation consisting of:
Tropicamide: 0.00016g
Phenylephrine hydrochloride: 0.00258g
Lidocaine hydrochloride: 0.00943g
Water for injectable Q.S. for a 1 ml bottle for single use.
